# EUROPEAN PATENT SPECIFICATION

(11) **EP 4 311 500 B1**
(45) Date of publication and mention of the grant of the patent: **03.06.2026**
(21) Application number: 22187632.9
(22) Date of filing: 28.07.2022
(51) Int. Cl.: A61B 8/00

(54) **METHOD, SYSTEM, AND COMPUTER PROGRAM FOR PERFORMING PERIODONTAL MEASUREMENTS FROM ULTRASOUND IMAGES**
VERFAHREN, SYSTEM UND COMPUTERPROGRAMM ZUR DURCHFÜHRUNG VON PERIODONTALEN MESSUNGEN AUS ULTRASCHALLBILDERN
PROCÉDÉ, SYSTÈME ET PROGRAMME INFORMATIQUE PERMETTANT D'EFFECTUER DES MESURES PARODONTALES À PARTIR D'IMAGES ÉCHOGRAPHIQUES

(43) Date of publication of application: 31.01.2024
(73) Proprietor: Carestream Dental LLC, Atlanta, GA 30339 (US); TROPHY SAS, 77435 Marne la Vallee Cedex 2 (FR)
(72) Inventor: GILLIBERT, Luc, 77435 MARNE LA VALLEE CEDEX 2 (FR); CAPRI, Arnaud, 77435 MARNE LA VALLEE CEDEX 2 (FR)
(74) Representative: Casalonga

(56) References cited:
- WO-A2-2006/105476
- US-A1- 2019 142 555
- US-A1- 2022 110 605
- US-A1- 2022 151 756
- US-A1- 2022 202 394

## Description

### FIELD OF THE INVENTION

The present invention relates to the field of intraoral measurement methods and devices for the health care industry. Particularly, but not exclusively, the invention relates to a method, a system, and a computer program for performing periodontal measurements from images acquired by ultrasound sensors, such as periodontal pocket depth measurements.

### BACKGROUND OF THE INVENTION

An important measure of gum health is the depth of a periodontal pocket located around each tooth. The space between the gum and the tooth before it attaches deepens in the presence of gum disease. To monitor and evaluate gum health, the pocket depth must be measured, recorded, and monitored over time. In a healthy mouth, a pocket can be anywhere from 1 to 3 millimeters deep (under 3 mm deep a pocket is considered healthy, while 4 mm or deeper it is considered unhealthy).

A graduated periodontal probe may be used to measure the depth of a periodontal pocket, i.e. to measure the distance from the top of the periodontal pocket to the bottom of the periodontal pocket where the tissue is connected through ligaments to the root.

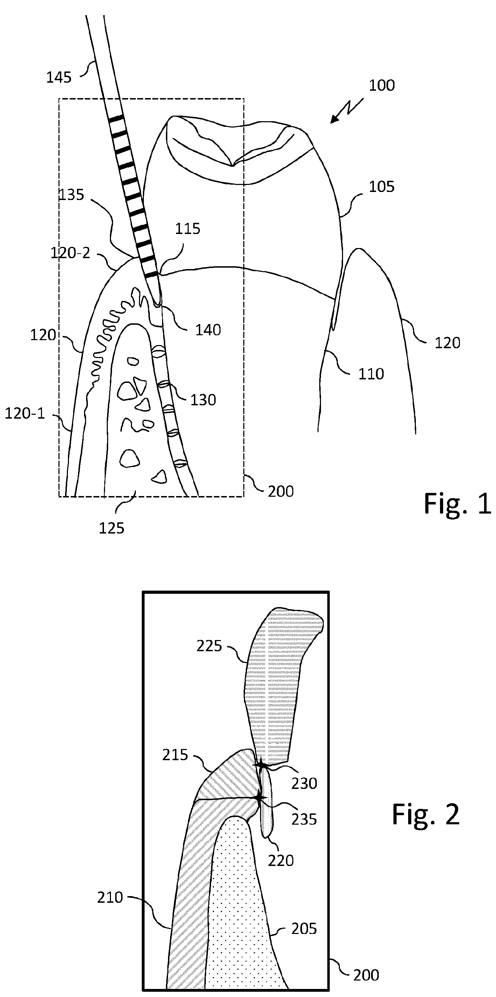

**Figure 1** illustrates schematically a sectional view of a portion of a tooth and a jaw (for the sake of clarity, only the structure located on the left part of the tooth is detailed).

As illustrated, tooth 100 comprises enamel 105 and cementum 110 that meets along a line 115 denoted CEJ (cementum-enamel junction). Cementum is the calcified tissue that forms the outer envelope of the tooth root. It represents the dental anchor point of the desmodontal fibers of the alveolar-dental ligament.

The jaw comprises gingiva 120, alveolar bone 125, and alveolar ligament (or desmodont) 130.

Gingiva 120 is a richly vascularized mucous membrane covering the bone and normally attaching to the root of the tooth by forming a slight fold called the gingival sulcus or periodontal pocket. Accordingly, gingiva 120 comprises a gingiva portion 120-1 attached to the alveolar bone and a free gingiva portion 120-2. The alveolar ligament (or desmodont) is made up of fibers that allow the attachment of the tooth to the alveolar bone.

Measuring a periodontal pocket aims at determining the height between the end of the free gingiva portion and the bottom of the periodontal pocket, for example the height between the end of the free gingiva portion referenced 135 and the bottom of the periodontal pocket referenced 140. As illustrated, such a height may be measured using graduated periodontal probe 140, by inserting the probe to the bottom of the cavity. When carried out by an experienced practitioner, the obtained measures are accurate.

However, while measuring a periodontal pocket with a graduated periodontal probe is generally efficient, this task is very unpleasant for the patient and time consuming for the practitioner because for each tooth several measurements (e.g. 6 measurements) have to be made and noted or recorded.

To cope with such drawbacks, ultrasound imaging has been adapted for intraoral use in a number of implementations and has been found to have particular utility for tasks such as measurement of periodontal pocket depth. Conditions such as gingivitis, for example, can be detected by sensing the acoustic response of tissues.

Because of the non-emission of ionizing radiation, ultrasound imaging is inherently safer than ionizing methods and also allows the repeatability of the examination if needed. Ultrasound imaging can be used as a substitute for, or a complement to, various types of radiography (cone beam computed tomography or CBCT, panoramic x-ray, or intraoral x-ray imaging), magnetic resonance imaging (MRI), or nuclear medicine.

Ultrasound imaging may use high-frequency sound waves, typically between 1 to 100MHz. High frequency waves being more attenuated than low-frequency waves for a given distance, high-frequency waves are suitable mainly for imaging superficial structures, e.g. for dermatology, or dental imaging. For example, high-frequency sound waves may preferably be between 10 to 50 MHz for periodontal pocket investigation. Conversely, low-frequency waves are suitable for imaging the deepest structures of the body.

An ultrasound imaging apparatus generally comprises one or several transducers that act as ultrasound beam emitters and/or ultrasound beam receivers to receive echoes from the emitted signals. In addition, the ultrasound imaging apparatus may comprise various processing and display components used for generating and presenting images from acquired signals. An ultrasound beam emitter generates an ultrasound signal from an electrical signal and conversely, an ultrasound receiver generates electrical pulses from a mechanical ultrasound signal.

Objects in the path of emitted ultrasound signals return a portion of the ultrasound energy back to the transducer which generates electrical signals indicative of the detected structures. The electrical signals generated from the received ultrasound signal can be delayed for selected times specific to each transducer, so that ultrasonic energy scattered from selected regions adds coherently, while ultrasonic energy from other regions has no perceptible impact. Further, the emission of ultrasound signals can be delayed in order to enable adaptive focusing. The electronic adaptive focusing makes it possible to increase the resolution depending on the depth of the imaged organ.

Array processing techniques used for generating and processing received signals in this way are termed "beamforming".

Particular challenges with intraoral ultrasound imaging relate to interpreting the images that are generally gray level images from which it is difficult to recognize the different parts of a patient's mouth and thus, to carry out certain measurements such as determining the height of a periodontal pocket. In addition, the ultrasound probe used to obtain ultrasound images should be correctly oriented and positioned to obtain reliable measurements.

US 2022/110605 is directed to a method of detecting an abnormality along a portion of a dental arch. An ultrasound probe is moved along the dental arch and an emitted ultrasound signal and the corresponding echoed signal are used to build ultrasound images from which abnormalities may be detected. The built images may be filtered to use only the images comprising features of interest.

US 2022/202394 discloses a wireless portable ultrasound imaging apparatus. The ultrasound signals are used to create images that may be displayed on the screen of a smart tablet or phone to carry out some measurements.

Therefore, there is a need for a method, a system, and a computer program making it possible to obtain automatically certain features of a patient's mouth, in particular to obtain automatically the depth of periodontal pockets and/or the distance between the epithelial attachment and the cementum-enamel junction (CEJ).

### SUMMARY OF THE INVENTION

The present invention has been devised to address one or more of the foregoing concerns.

In this context, there is provided a method, a system, and a computer program making it possible to obtain automatically certain features of a patient's mouth from ultrasound images of the patient's mouth, without analysis of these ultrasound images by a practitioner.

According to an aspect of the invention, there is provided a method of carrying out periodontal measurements from ultrasound images according to claim 1 comprising:
obtaining at least one ultrasound image of a jaw portion comprising at least one tooth periodontium,
generating a plurality of images from the at least one obtained ultrasound image, each of the generated images representing a presence of at least one anatomical periodontal feature in the at least one obtained ultrasound image,
validating the presence of at least one anatomical periodontal feature in the at least one obtained ultrasound image from the corresponding generated image,
if the presence of anatomical periodontal features requested for carrying out at least one periodontal measurement, in the at least one obtained ultrasound image, is validated, carrying out the at least one periodontal measurement from anatomical periodontal features which presence is validated, and
displaying, storing, or transmitting a result of the at least one periodontal measurement.

The method according to the invention makes it possible to provide automatically some periodontal measurements from ultrasound images of a tooth periodontium, without requiring any skill regarding interpretation of ultrasound images, allowing a practitioner to focus on handling a sensor probe and on a patient's mouth.

According to some embodiments of the invention, the at least one periodontal measurement comprises at least one of Clinical Attachment Level, CAL, measurement, a pocket depth, PD, measurement, and a Gingival Margin, GM, measurement.

Still according to some embodiments of the invention, the method further comprises, if it is determined that the at least one obtained ultrasound image does not comprise anatomical periodontal features requested for carrying out the at least one periodontal measurement, obtaining at least one other ultrasound image of a jaw portion comprising at least one tooth periodontium and repeating the generating and validating using the at least one other ultrasound image.

Still according to some embodiments of the invention, the method further comprises, if it is determined that the at least one obtained ultrasound image does not comprise anatomical periodontal features, displaying a message indicating that the at least one periodontal measurement cannot be carried out from the at least one obtained ultrasound image.

Still according to some embodiments of the invention, the validating comprises determining, in the generated image, a number of pixels whose value is equal to or greater than a first threshold, and comparing the determined number of pixels with a second threshold.

Still according to some embodiments of the invention, the method further comprises identifying a structure in at least one of the generated images, the structure being identified as a function of a position of pixels whose value is equal to or greater than a third threshold, carrying out the at least one periodontal measurement being based on the identified structure.

Still according to some embodiments of the invention, the method further comprises identifying a landmark of interest in at least one of the generated images, the landmark of interest being identified as a function of a predetermined number of pixels whose values are the highest, carrying out the at least one measurement being based on the identified landmark.

Still according to some embodiments of the invention, the method further comprises downscaling the at least one obtained image, the generating being based on the at least one downscaled obtained image.

Still according to some embodiments of the invention, the generating is carried out using a deep neural network.

Still according to some embodiments of the invention, the deep neural network is a convolutional neural network of a U-net type.

Still according to some embodiments of the invention, the method further comprises lowering a coding bit depth of weight of the deep neural network after training of the deep neural network.

Still according to some embodiments of the invention, the obtained at least one ultrasound image belongs to a plane comprising a dental axis of the tooth and at least one point of the tooth surface belonging to the tooth periodontium.

Still according to some embodiments of the invention, the at least one anatomical periodontal feature comprises an enamel, cementum, a gingiva, an alveolar bone, a cementum-enamel junction, and/or an epithelial attachment.

According to other aspects of the invention, there is provided a device comprising a processing unit configured for carrying out each step of the method described above. The other aspects of the present disclosure have advantages similar to the one above-mentioned aspect.

According to some embodiments of the invention, the processing unit comprises a specific integrated circuit embedding a deep neural network, the deep neural network being used to generate images each of which representing a presence of at least one anatomical periodontal feature in the at least one obtained ultrasound image.

At least parts of the methods according to the invention may be computer implemented. Accordingly, the present invention may take the form of an entirely hardware embodiment, an entirely software embodiment (including firmware, resident software, micro-code, etc.) or an embodiment combining software and hardware aspects that may all generally be referred to herein as a "circuit", "module" or "system". Furthermore, the present invention may take the form of a computer program product embodied in any tangible medium of expression having computer usable program code embodied in the medium.

Since the present invention can be implemented in software, the present invention can be embodied as computer readable code for provision to a programmable apparatus on any suitable carrier medium. A tangible carrier medium may comprise a storage medium such as a floppy disk, a CD-ROM, a hard disk drive, a magnetic tape device or a solid-state memory device and the like. A transient carrier medium may include a signal such as an electrical signal, an electronic signal, an optical signal, an acoustic signal, a magnetic signal or an electromagnetic signal, e.g. a microwave or RF signal.

### BRIEF DESCRIPTION OF THE DRAWINGS

Embodiments of the invention will now be described, by way of example only, and with reference to the following drawings in which:
**Figure 1** illustrates schematically a portion of a tooth and a jaw;
**Figure 2** illustrates an example of detected structures and identified landmarks in an ultrasound image, making it possible to measure reliably the depth of periodontal pockets;
**Figure 3** illustrates an example of steps of a method for carrying out periodontal measurements from ultrasound images, for example to provide measurements of the depth of periodontal pockets, according to some embodiments of the invention;
**Figure 4** illustrates an example of a deep neural network that can be used to process downscaled ultrasound images so as to detect structures such as an alveolar bone, a gingiva portion attached to the alveolar bone, a free gingiva portion, cementum, and enamel and to identify landmarks such as a cementum-enamel junction and an epithelial attachment; and
**Figure 5** is a schematic block diagram of a computing device for implementation of one or more embodiments of the invention.

### DETAILED DESCRIPTION OF THE INVENTION

The following is a detailed description of particular embodiments of the invention, reference being made to the drawings in which the same reference numerals identify the same elements of structure in each of the Figures.

In the drawings and text that follow, like elements are designated with like reference numerals, and similar descriptions concerning elements and an arrangement or interaction of elements already described are omitted. Where they are used, the terms "first", "second", and so on, do not necessarily denote any ordinal or priority relation, but may simply be used to more clearly distinguish one element from another, unless specified otherwise.

In the context of the present disclosure, the terms "viewer", "operator", and "user" are considered to be equivalent and refer to the viewing practitioner, technician, or other person who acquires, views, and manipulates an ultrasound image, such as a intraoral image, on a display monitor. An "operator instruction," "user instruction," or "viewer instruction" is obtained from explicit commands entered by the viewer, such as by clicking a button on the ultrasound probe or system hardware or by using a computer mouse or by using a touch screen or a keyboard entry.

In the context of the present disclosure, the phrase "in signal communication" indicates that two or more devices and/or components are capable of communicating with each other via signals that travel over some type of signal path. Signal communication may be wired or wireless. The signals may be communication, power, data, or energy signals. The signal paths may include physical, electrical, magnetic, electromagnetic, optical, wired, and/or wireless connections between the first device and/or component and second device and/or component. The signal paths may also include additional devices and/or components between the first device and/or component and second device and/or component.

The term "subject" refers to the gums and other intraoral soft tissues (and possibly to tooth surfaces) of a patient that is being imaged and, in optical terms, can be considered equivalent to the "object" of the corresponding imaging system.

According to some embodiments of the invention, the depth of periodontal pockets is measured automatically from ultrasound images. When the device producing the images is not correctly positioned (i.e. when the obtained images do not make it possible to measure reliably the depth of the periodontal pockets), measurements are not carried out and an error message is provided. Measurements are based on detection of particular materials or tissues in the ultrasound images and on identification of particular landmarks (or specific anatomical points) such as epithelial attachment and/or cementum-enamel junction. Such detection and identification are carried out in real time making it possible, for example, to process at least ten images per second. Since measurements of the depth of the periodontal pockets are carried out automatically, there is no need to display the ultrasound images from which measurements are obtained.

According to some particular embodiments, at least some of the following structures and landmarks are detected and identified to make it possible to measure reliably the depth of periodontal pockets:
- the enamel;
- the cementum;
- the gingiva;
- the alveolar bone;
- the cementum-enamel junction; and/or
- the epithelial attachment.

According to some embodiments, machine learning is used for detecting the anatomical structures and landmarks relevant to carry out the desired measurements. To that end, the system is able to determine whether the structures and landmarks are present in the image (classification) and if they are present, the system locates them precisely (segmentation or landmark localization). Next, from these structures and landmarks, the system carries out the measurements, for example periodontal measurements.

**Figure 2** illustrates an example of detected structures and identified landmarks in an ultrasound image, making it possible to measure reliably the depth of periodontal pockets. For the sake of illustration, the ultrasound image used represents a portion of the tooth and jaw represented in Figure 1, as represented with reference 200.

After having processed ultrasound image 200, the alveolar bone, the gingiva portion attached to the alveolar bone, the free gingiva portion, the cementum, and the enamel, referenced 205, 210, 215, 220, and 225, respectively, are detected and located. Likewise, the cementum-enamel junction and the epithelial attachment, referenced 230 and 235, respectively, are identified and located.

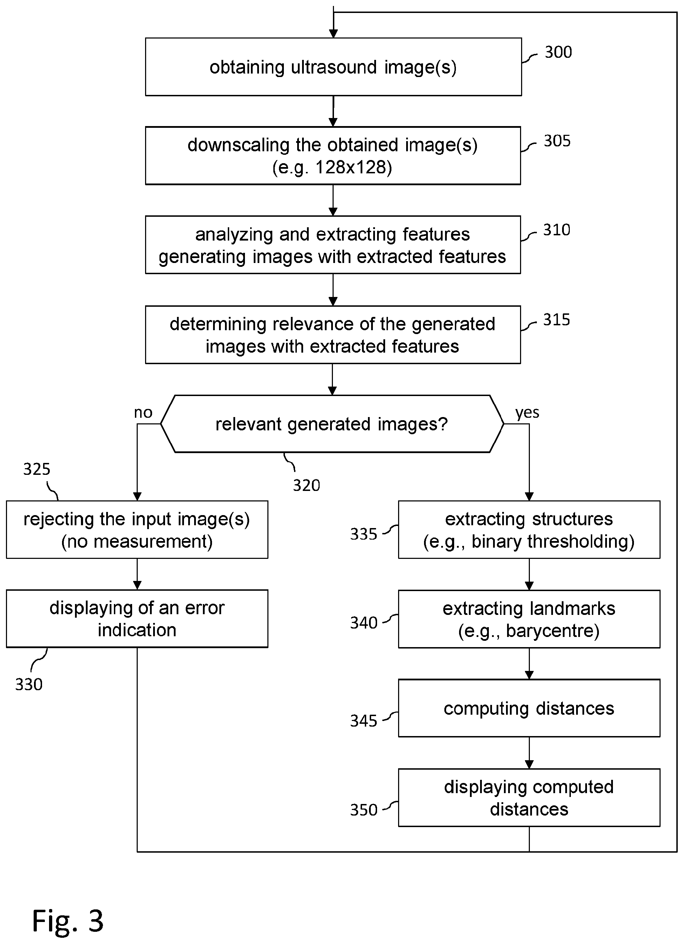

**Figure 3** illustrates an example of steps of a method for carrying out periodontal measurements from ultrasound images, for example to provide measurements of the depth of periodontal pockets, according to some embodiments of the invention.

As illustrated, a first step is directed to obtaining one or several images (step 300), for example 2D (two-dimensional) images resulting from data acquired from a 2D ultrasound probe, denoted ultrasound images. The obtained image should be taken from a viewing angle making it possible to detect the searched for structure and the searched for landmarks of interest. Accordingly, the obtained images preferably belong to a plane comprising the dental axis of the tooth and at least one point of the tooth surface belonging to the tooth periodontium.

Next, the obtained ultrasound images are downscaled to a resolution allowing a fast and accurate detection of structures and/or of landmarks of interest (step 305), for example a resolution of 128x128 pixels. The downscaled images are then processed to analyze and extract features corresponding to searched for structures or enabling identification of landmarks of interest (step 310). According to some embodiments of the invention, this step involves a deep neural network such as a convolutional neural network which generates several result images, for example six images (one for each searched for anatomical landmark and structure: the enamel, the cementum, the gingiva, the alveolar bone, the cementum-enamel junction, and the epithelial attachment).

Next, each of the obtained images is analyzed, for example using appropriate thresholds, to determine whether it is relevant for carrying out periodontal measurements (step 315). Such analysis may consist in comparing the value of each pixel of the considered generated image with a first threshold and in comparing the number of pixels whose value is equal to or greater than the first threshold with a second threshold. The value of the first and the second thresholds may be predetermined or determined dynamically. For the sake of illustration, they may be determined automatically during the training of the deep neural networks used to analyze and extract features and to generate images.

It is noted here that the process making it possible to determine whether a generated image is relevant for carrying out periodontal measurements may apply to a single input ultrasound image or to several input ultrasound images (preferably contiguous images), for example to take into account small movements of the probe (which may improve the relevance of the data in the input ultrasound images). According to some embodiments, one of several generated images corresponding to the same structure or the same landmark of interest is selected on the basis of the number of pixels whose value is equal to or greater than the first threshold (e.g., the generated image associated with the highest number of pixels whose value is equal to or greater than the first threshold is selected).

If the number of pixels whose value is equal to or greater than the first threshold is lower than the second threshold, it means that the searched for structure or the searched for landmark of interest is absent or that the probe is misplaced. Accordingly, it is concluded that the input ultrasound image is not usable to make periodontal measurements and thus, it is rejected (step 325). According to some embodiments, an indication that the input images are rejected is displayed (step 330) for indicating to the practitioner that the images cannot be used to carry out periodontal measurements. From such an indication, the practitioner may understand that the probe is not correctly positioned and thus, may move it until the desired measurements are obtained.

As illustrated, the algorithm may then loop to step 300 in order to process new ultrasound images.

It is noted here that rejecting images which do not seem to contain relevant data avoids losing processing time in processing meaningless data, thus enabling high frame rate performance.

On the contrary, if the number of pixels whose value is equal to or greater than the first threshold is equal to or greater than the second threshold, preferably for each of the generated images corresponding to the considered input ultrasound image, it is concluded that the latter contains relevant information from which periodontal measurements may be carried out.

Accordingly, the searched for structures are identified (step 335), for example by converting the corresponding generated images into binary images. For the sake of illustration, the searched for structures may correspond to the areas of the generated images wherein the value of the pixels is equal to or greater than a third threshold. The value of the third threshold may be predetermined or determined dynamically. It may be equal to the first threshold. From the coordinates of these points in the generated images (which are the same as in the downscaled images), it is possible to compute the position of the searched for structures in the ultrasound images (based on the parameters of the downscaling operation).

Next or in parallel, the landmarks of interest (e.g., the CEJ and the epithelial attachment) are extracted from the two corresponding generated images (step 340). To that end, a determined number of the pixels (K pixels) with the highest value in these two images are selected and used to compute two reference points (one per generated image). Such reference points may be, for example, the barycenter of the selected points in each of the two generated images. For the sake of illustration, K may be chosen in the range from 2 to 50. For example, K may be set to 10. These two barycenters correspond to the position of the CEJ and the epithelial attachment.

From the coordinates of these points in the generated images (which are the same as in the downscaled images), it is possible to compute the position of the CEJ and of the epithelial attachment in the original image (based on the parameters of the downscaling operation).

Next, using the positions of the searched for structures and of the searched for landmarks in the input images, periodontal measurements may be carried out (step 345). For the sake of illustration, at least some of the following measurements may be carried out:
- the distance denoted the Clinical Attachment Level (CAL) which corresponds to the distance between the CEJ and the epithelium attachment,
- the Pocket Depth (PD) which corresponds to the distance between the top of the free gingiva projected preferably orthogonally to the tooth axis on the tooth surface and the epithelium attachment, and/or
- the Gingival Margin (GM) which corresponds to the distance between the top of the free gingiva projected preferably orthogonally to the tooth axis on the tooth surface and the CEJ.

The computed distances are displayed (step 350). According to some embodiments, the ultrasound images used to measure these distances are not displayed. Whatever the orientation of the probe, as soon as minimal periodontium structures are detected in the image, the algorithm provides measurements.

As illustrated, the algorithm may then loop to step 300 in order to process new ultrasound images.

It is noted here that although the illustrated example is based on the analysis of 2D ultrasonic images, for example taken at a frame rate of ten images per second, a 3D approach can be considered by using 3D volumes obtained either by a 3D probe directly capturing thick layers of the jaw (e.g., by using multilayer transducers) or by reconstructing 3D volumes from several 2D images as the input of the deep neural network.

As disclosed above and according to some particular embodiments, deep neural networks are used to process downscaled ultrasound images so as to detect searched for structures and to identify searched for landmarks. Such deep neural networks may be derived from the convolutional neural networks known as U-Net. The U-Net network comprises a contracting path and an expansive path. The contracting path is a typical convolutional network that consists of repeated application of convolutions, each followed by a rectified linear unit (ReLU) and a max pooling operation. During the contraction, the spatial information is reduced while feature information is increased. The expansive pathway combines the feature and spatial information through a sequence of up-convolutions and concatenations with high-resolution features from the contracting path. Artificial neural networks of the U-net type are described, for example, in the article entitled "U-net: Convolutional networks for biomedical image segmentation", Ronneberger, O., Fischer, P. & Brox, T., Medical Image Computing and Computer-Assisted Intervention - MICCAI 2015. Lecture Notes in Computer Science, 9351, 234-241 (Springer International Publishing, 2015).

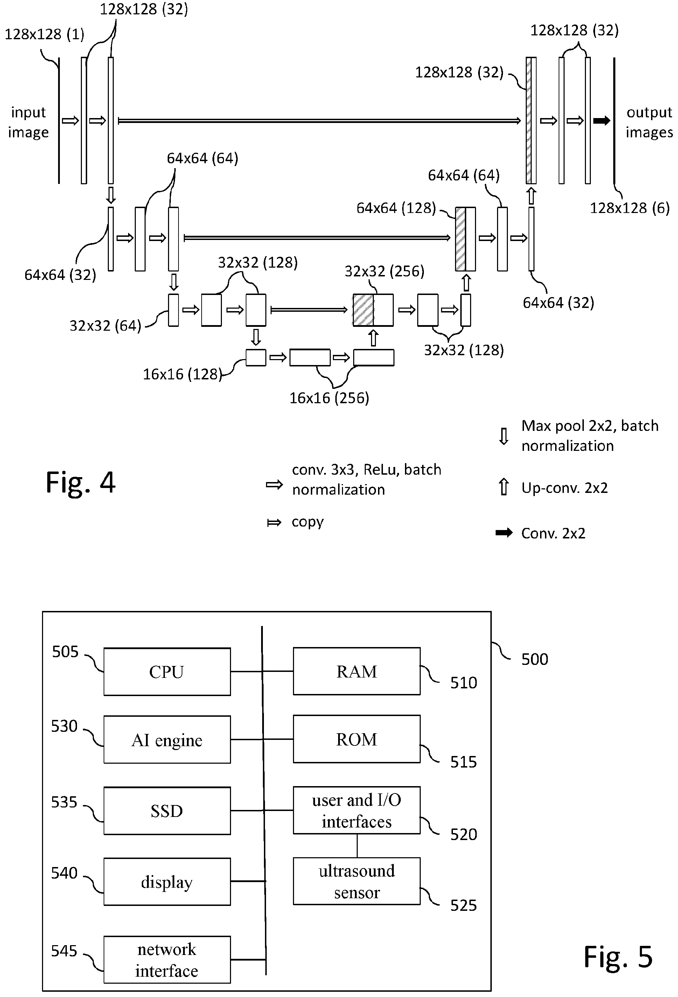

**Figure 4** illustrates an example of a deep neural network that can be used to process ultrasound images, preferably downscaled ultrasound images, so as to detect structures such as an alveolar bone, a gingiva portion attached to the alveolar bone, a free gingiva portion, cementum, and enamel and to identify landmarks such as a cementum-enamel junction and an epithelial attachment.

According to the illustrated example, the input of the deep neural network is a single image having 128 x 128 pixels, that may be coded on one byte each, and the output comprises six images having 128 x 128 pixels, that may also be coded on one byte each. Each box represents a multi-channel feature map. The x and y size of the feature map is given by the first two numbers of the label associated with the box and the number of channels is provided with the third number (between brackets). The crosshatched boxes represent copied feature maps and the arrows denote the operations as indicated in Figure 4.

As illustrated, the architecture of the deep neural network comprises a contracting path (left side) and an expansive path (right side). The contracting path aims at applying several times a double 3x3 convolution, followed by a rectified linear unit (ReLU) and a 2x2 max pooling operation with stride 2 for down-sampling. At each down-sampling step, the number of feature channels is doubled. Conversely, the expansive path aims at up-sampling of a feature map, which is followed by a 2x2 convolution ("up-convolution") that halves the number of feature channels, a concatenation with the correspondingly cropped feature map from the contracting path, and two 3x3 convolutions, each followed by a ReLU. At the final layer, a 1x1 convolution is used to map each 32-component feature vector to the 6 classes, each representing the enamel, the cementum, the gingiva, the alveolar bone, the cementum-enamel junction, or the epithelial attachment, making it possible to detect and locate the six anatomic structures and landmarks in a single pass.

According to some embodiments, the deep neural network illustrated in Figure 4 is trained with real-life data where the correct structures and landmarks have been determined by experts. For the sake of illustration, a first database with 950 images (where all the anatomic structures and landmarks are present and identified) and a second database comprising 400 images without anatomic structures and/or landmarks, taken when the probe was badly positioned, were created. These two databases were split in a training set, a testing set, and a validation set and used for training.

After training the deep neural network, the coding bit depth of the parameters of the deep neural network, in particular the weight of the neurons, is preferably modified to reduce the size of the deep neural network while maintaining a good level of accuracy and precision. For example, the coding bit depth may consist in coding the weight of the neurons on one byte, making it possible to run the deep neural network on dedicated hardware, such as an Edge TPU (a Google's purpose-built ASIC designed to run Al engines in embedded systems).

It is noted that while the deep neural network illustrated in Figure 4 has proven its effectiveness, some parameters may be changed, such as the size of the feature maps and/or the number of channels.

**Figure 5** is a schematic block diagram of computing device for implementation of one or more embodiments of the invention, in particular for carrying out the steps or parts of the steps described by reference to Figure 3.

Computing device 500 comprises a communication bus that may be connected to all or some of the following elements:
- a central processing unit 505, such as a microprocessor, denoted CPU;
- a random access memory 510, denoted RAM, for storing the executable code of the method of some embodiments of the invention as well as the registers adapted to record variables and parameters necessary for implementing a method for carrying out periodontal measurements from ultrasound images according to some embodiments of the invention, the memory capacity of which can be expanded by an optional RAM connected to an expansion port for example;
- a read-only memory 515, denoted ROM, for storing computer programs for implementing some embodiments of the invention;
- a user interface and/or an input/output interface 520 which can be used for receiving inputs from a user, providing information to a user, and/or receiving/sending data from/to internal sensors and/or external devices, in particular receiving data from a sensor such as ultrasound sensor 525, which may be embedded within computing device 500 or which may be an external device connected to computing device 500 via wires or via a wireless link; and
- an Al engine 530 which is configured for analyzing ultrasound images or downscaled ultrasound images and for generating images representing searched for structures and/or searched for landmarks of interest. It is noted that the Al engine may be embedded within computing device 500, may be in a remote processing unit, or may be spread over one or more servers, for example in a cloud.

Optionally, the communication bus of computing device 500 may be connected to a solid-state disk 535 denoted SSD (or a hard disk) used as a mass storage device and/or a display 540, for example for displaying periodontal measurements.

The communication bus of computing device 500 may also be connected to a network interface 545 typically connected to a communication network over which digital data can be transmitted or received for receiving/sending data from/to remote devices, in particular to a dental information system and/or storage device 535. The network interface 545 can be a single network interface, or composed of a set of different network interfaces (for instance wired and wireless interfaces, or different kinds of wired or wireless interfaces). Data packets are written to the network interface for transmission or are read from the network interface for reception under the control of the software application running in the CPU 505

The executable code may be stored either in read-only memory 515, on solid state device 535 or on a removable digital medium such as for example a memory card. According to a variant, the executable code of the programs can be received by means of a communication network, via the network interface 534, in order to be stored in one of the storage means of the computing device 500, such as solid-state device 535, before being executed.

Central processing unit 505 is adapted to control and direct the execution of the instructions or portions of software code of the program or programs according to some embodiments of the invention, the instructions being stored in one of the aforementioned storage means. After powering on, CPU 505 is capable of executing instructions from main RAM memory 510 relating to a software application after those instructions have been loaded from ROM 515 or from solid-state device 535 for example. Such a software application, when executed by CPU 505, causes the steps herein disclosed to be performed.

Any step herein disclosed may be implemented in software by execution of a set of instructions or program by a programmable computing machine, such as a PC ("Personal Computer"), a DSP ("Digital Signal Processor") or a microcontroller; or else implemented in hardware by a machine or a dedicated component, such as an FPGA ("Field-Programmable Gate Array") or an ASIC ("Application-Specific Integrated Circuit").

Although the present disclosure has been described herein above with reference to some specific embodiments, the present invention is not limited to these specific embodiments, and modifications will be apparent to a person skilled in the art which lie within the scope of the present invention.

Many further modifications and variations will suggest themselves to those versed in the art upon making reference to the foregoing illustrative embodiments, which are given by way of example only and which are not intended to limit the scope of the invention, that being determined solely by the appended claims. In particular, the different features from different embodiments may be interchanged, where appropriate.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used.

## Claims

1. A method of carrying out periodontal measurements from ultrasound images performed by a processing unit comprising:
obtaining (300) at least one ultrasound image of a jaw portion comprising at least one tooth periodontium,
generating (310) a plurality of images from each of the obtained ultrasound image, each of the generated images representing a presence of at least one anatomical periodontal feature in the at least one obtained ultrasound image,
validating the presence of at least one anatomical periodontal feature in the at least one obtained ultrasound image from the corresponding generated image,
if the presence of anatomical periodontal features requested for carrying out at least one periodontal measurement, in the at least one obtained ultrasound image, is validated (320), carrying out (345) the at least one periodontal measurement from anatomical periodontal features which presence is validated, and
displaying, storing, or transmitting a result of the at least one periodontal measurement.

2. The method of claim 1, wherein the at least one periodontal measurement comprises at least one of Clinical Attachment Level, CAL, measurement, a pocket depth, PD, measurement, and a Gingival Margin, GM, measurement.

3. The method of claim 1 or claim 2, further comprising, if it is determined that the at least one obtained ultrasound image does not comprise anatomical periodontal features requested for carrying out the at least one periodontal measurement, obtaining at least one other ultrasound image of a jaw portion comprising at least one tooth periodontium and repeating the generating and validating using the at least one other ultrasound image.

4. The method of any one of claims 1 to 3, wherein the validating comprises determining, in the generated image, a number of pixels whose value is equal to or greater than a first threshold, and comparing the determined number of pixels with a second threshold.

5. The method of claim 4, further comprising identifying (335) a structure in at least one of the generated images, the structure being identified as a function of a position of pixels whose value is equal to or greater than a third threshold, carrying out the at least one periodontal measurement being based on the identified structure.

6. The method of any one of claims 1 to 5, further comprising identifying (340) a landmark of interest in at least one of the generated images, the landmark of interest being identified as a function of a predetermined number of pixels whose values are the highest, carrying out the at least one measurement being based on the identified landmark.

7. The method of any one of claims 1 to 6, further comprising downscaling (305) the at least one obtained image, the generating being based on the at least one downscaled obtained image.

8. The method of any one of claims 1 to 7, wherein the generating is carried out using a deep neural network.

9. The method of claim 8, wherein the deep neural network is a convolutional neural network of a U-net type.

10. The method of claim 8 or claim 9, further comprising lowering a coding bit depth of weight of the deep neural network after training of the deep neural network.

11. The method of any one of claims 1 to 10, wherein the obtained at least one ultrasound image belongs to a plane comprising a dental axis of the tooth and at least one point of the tooth surface belonging to the tooth periodontium.

12. The method of any one of claims 1 to 11, wherein the at least one anatomical periodontal feature comprises an enamel, cementum, a gingiva, an alveolar bone, a cementum-enamel junction, and/or an epithelial attachment.

13. A computer program product for a programmable apparatus, the computer program product comprising a sequence of instructions for implementing each of the steps of the method according to any one of claims 1 to 12 when loaded into and executed by the programmable apparatus.

14. A device comprising a processing unit configured for carrying out each of the steps of the method according to any one of claims 1 to 12.

15. The device of claim 14, wherein the processing unit comprises a specific integrated circuit embedding a deep neural network, the deep neural network being used to generate images each of which representing a presence of at least one anatomical periodontal feature in the at least one obtained ultrasound image.

## Patentansprüche

1. Verfahren zum Vornehmen parodontaler Messungen aus Ultraschallbildern, die von einer Verarbeitungseinheit durchgeführt werden, umfassend:
Erhalten (300) von mindestens einem Ultraschallbild eines Kieferabschnitts, der mindestens ein Zahnparodontium umfasst,
Erzeugen (310) einer Vielzahl von Bildern aus jedem der erhaltenen Ultraschallbilder, wobei jedes der erzeugten Bilder ein Vorhandensein von mindestens einem anatomischen parodontalen Merkmal in dem mindestens einen erhaltenen Ultraschallbild darstellt,
Validieren des Vorhandenseins von mindestens einem anatomischen parodontalen Merkmal in dem mindestens einen aus dem entsprechenden erzeugten Bild erhaltenen Ultraschallbild,
wenn das Vorhandensein von anatomischen parodontalen Merkmalen, die für das Vornehmen mindestens einer parodontalen Messung angefordert werden, in dem mindestens einen erhaltenen Ultraschallbild validiert (320) ist, Vornehmen (345) der mindestens einen parodontalen Messung anhand anatomischer parodontaler Merkmale, deren Vorhandensein validiert ist, und
Anzeigen, Speichern oder Übertragen eines Ergebnisses der mindestens einen parodontalen Messung.

2. Verfahren nach Anspruch 1, wobei die mindestens eine parodontale Messung mindestens eine Clinical Attachment-Level-, CAL-,Messung, eine Taschentiefen-, PD-,Messung und eine Zahnfleischrand-, GM-,Messung umfasst.

3. Verfahren nach Anspruch 1 oder Anspruch 2, ferner umfassend, wenn bestimmt wird, dass das mindestens eine erhaltene Ultraschallbild keine anatomischen parodontalen Merkmale umfasst, die für das Vornehmen der mindestens einen parodontalen Messung angefordert werden, das Erhalten von mindestens einem weiteren Ultraschallbild eines Kieferabschnitts, der mindestens ein Zahnparodontium umfasst, und die Wiederholung des Erzeugens und Validierens unter Verwendung des mindestens einen weiteren Ultraschallbildes.

4. Verfahren nach einem der Ansprüche 1 bis 3, wobei das Validieren das Bestimmen einer Anzahl von Pixeln im erzeugten Bild, deren Wert gleich oder größer ist als ein erster Schwellenwert, und das Vergleichen der ermittelten Anzahl von Pixeln mit einem zweiten Schwellenwert umfasst.

5. Verfahren nach Anspruch 4, ferner umfassend das Identifizieren (335) einer Struktur in mindestens einem der erzeugten Bilder, wobei die Struktur als Funktion einer Position von Pixeln identifiziert wird, deren Wert gleich oder größer ist als ein dritter Schwellenwert, wobei das Vornehmen der mindestens einen parodontalen Messung auf der Grundlage der identifizierten Struktur erfolgt.

6. Verfahren nach einem der Ansprüche 1 bis 5, ferner umfassend das Identifizieren (340) eines Orientierungspunkts von Interesse in mindestens einem der erzeugten Bilder, wobei der Orientierungspunkt von Interesse als Funktion einer vorbestimmten Anzahl von Pixeln, deren Werte am höchsten sind, identifiziert wird, wobei das Vornehmen der mindestens einen Messung auf der Grundlage des identifizierten Orientierungspunkts erfolgt.

7. Verfahren nach einem der Ansprüche 1 bis 6, ferner umfassend das Herunterskalieren (305) des mindestens einen erhaltenen Bildes, wobei das Erzeugen auf der Grundlage des mindestens einen herunterskalierten erhaltenen Bildes erfolgt.

8. Verfahren nach einem der Ansprüche 1 bis 7, wobei das Erzeugen unter Verwendung eines tiefen neuronalen Netzwerks vorgenommen wird.

9. Verfahren nach Anspruch 8, wobei das tiefe neuronale Netzwerk ein konvolutionales neuronales Netzwerk vom U-Net-Typ ist.

10. Verfahren nach Anspruch 8 oder 9, ferner umfassend das Absenken einer Codierbittiefe des Gewichts des tiefen neuronalen Netzwerks nach dem Training des tiefen neuronalen Netzwerks.

11. Verfahren nach einem der Ansprüche 1 bis 10, wobei das erhaltene mindestens eine Ultraschallbild zu einer Ebene gehört, die eine Zahnachse des Zahns und mindestens einen Punkt der Zahnoberfläche umfasst, der zum Zahnparodontium gehört.

12. Verfahren nach einem der Ansprüche 1 bis 11, wobei das mindestens eine anatomische parodontale Merkmal einen Zahnschmelz, Zement, ein Zahnfleisch, einen Alveolarknochen, eine Zement-Schmelz-Grenze und/oder einen Epithelbefestigung umfasst.

13. Computerprogrammprodukt für eine programmierbare Einrichtung, wobei das Computerprogrammprodukt eine Abfolge von Anweisungen zum Umsetzen jedes der Schritte des Verfahrens nach einem der Ansprüche 1 bis 12 umfasst, wenn es auf die programmierbare Einreichtung geladen und von ihr ausgeführt wird.

14. Vorrichtung, die eine Verarbeitungseinheit umfasst, die zum Vornehmen jedes der Schritte des Verfahrens nach einem der Ansprüche 1 bis 12 konfiguriert ist.

15. Vorrichtung nach Anspruch 14, wobei die Verarbeitungseinheit eine spezifische integrierte Schaltung umfasst, die ein tiefes neuronales Netzwerk einbettet, wobei das tiefe neuronale Netzwerk verwendet wird, um Bilder zu erzeugen, von denen jedes ein Vorhandensein von mindestens einem anatomischen parodontalen Merkmal in dem mindestens einen erhaltenen Ultraschallbild darstellt.

## Revendications

1. Procédé de réalisation de mesures parodontales à partir d'images échographiques effectuées par une unité de traitement comprenant :
l'obtention (300) d'au moins une image échographique d'une partie de mâchoire comprenant au moins un parodonte dentaire,
la génération (310) d'une pluralité d'images à partir de chacune de l'image échographique obtenue, chacune des images générées représentant une présence d'au moins une caractéristique parodontale anatomique dans l'au moins une image échographique obtenue,
la validation de la présence d'au moins une caractéristique parodontale anatomique dans l'au moins une image échographique obtenue à partir de l'image générée correspondante,
si la présence de caractéristiques parodontales anatomiques demandées pour réaliser au moins une mesure parodontale, dans l'au moins une image échographique obtenue, est validée (320), la réalisation (345) de l'au moins une mesure parodontale à partir de caractéristiques parodontales anatomiques dont la présence est validée, et l'affichage, le stockage ou la transmission d'un résultat de l'au moins une mesure parodontale.

2. Procédé selon la revendication 1, dans lequel l'au moins une mesure parodontale comprend au moins l'une d'une mesure de niveau d'attache clinique, CAL, d'une mesure de profondeur de poche, PD, et d'une mesure de bord gingival, GM.

3. Procédé selon la revendication 1 ou la revendication 2, comprenant en outre, s'il est déterminé que l'au moins une image échographique obtenue ne comprend pas de caractéristiques parodontales anatomiques demandées pour réaliser l'au moins une mesure parodontale, l'obtention d'au moins une autre image échographique d'une partie de mâchoire comprenant au moins un parodonte dentaire et la répétition de la génération et de la validation à l'aide de l'au moins une autre image échographique.

4. Procédé selon l'une quelconque des revendications 1 à 3, dans lequel la validation comprend la détermination, dans l'image générée, d'un nombre de pixels dont la valeur est égale ou supérieure à un premier seuil, et la comparaison du nombre déterminé de pixels à un deuxième seuil.

5. Procédé selon la revendication 4, comprenant en outre l'identification (335) d'une structure dans au moins l'une des images générées, la structure étant identifiée en fonction d'une position de pixels dont la valeur est égale ou supérieure à un troisième seuil, la réalisation de l'au moins une mesure parodontale étant basée sur la structure identifiée.

6. Procédé selon l'une quelconque des revendications 1 à 5, comprenant en outre l'identification (340) d'un repère d'intérêt dans au moins l'une des images générées, le repère d'intérêt étant identifié en fonction d'un nombre prédéterminé de pixels dont les valeurs sont les plus élevées, la réalisation de l'au moins une mesure étant basée sur le repère identifié.

7. Procédé selon l'une quelconque des revendications 1 à 6, comprenant en outre la réduction (305) de l'au moins une image obtenue, la génération étant basée sur l'au moins une image obtenue réduite.

8. Procédé selon l'une quelconque des revendications 1 à 7, dans lequel la génération est réalisée à l'aide d'un réseau neuronal profond.

9. Procédé selon la revendication 8, dans lequel le réseau neuronal profond est un réseau neuronal à convolution d'un type U-net.

10. Procédé selon la revendication 8 ou la revendication 9, comprenant en outre l'abaissement d'une profondeur de bit de codage du poids du réseau neuronal profond après entraînement du réseau neuronal profond.

11. Procédé selon l'une quelconque des revendications 1 à 10, dans lequel l'au moins une image échographique obtenue appartient à un plan comprenant un axe dentaire de la dent et au moins un point de la surface dentaire appartenant au parodonte dentaire.

12. Procédé selon l'une quelconque des revendications 1 à 11, dans lequel l'au moins une caractéristique parodontale anatomique comprend un émail, du cément, une gencive, un os alvéolaire, une jonction émail-cément et/ou une attache épithéliale.

13. Produit programme informatique pour un appareil programmable, le produit programme informatique comprenant une séquence d'instructions pour mettre en œuvre chacune des étapes du procédé selon l'une quelconque des revendications 1 à 12 lorsqu'elles sont chargées dans et exécutées par l'appareil programmable.

14. Dispositif comprenant une unité de traitement configurée pour réaliser chacune des étapes du procédé selon l'une quelconque des revendications 1 à 12.

15. Dispositif selon la revendication 14, dans lequel l'unité de traitement comprend un circuit intégré spécifique intégrant un réseau neuronal profond, le réseau neuronal profond étant utilisé pour générer des images dont chacune représente une présence d'au moins une caractéristique parodontale anatomique dans l'au moins une image échographique obtenue.
